# EUROPÄISCHE PATENTSCHRIFT

(11) **EP 2 606 922 B1**
(45) Veröffentlichungstag und Bekanntmachung des Hinweises auf die Patenterteilung: **15.02.2017**
(21) Anmeldenummer: 11010187.0
(22) Anmeldetag: 23.12.2011
(51) Int. Cl.: A61M 5/145, A61M 5/168

(54) **Infusionspumpe mit Antriebsvorrichtung und Blockiereinrichtung für den Infusionsspritzenkolbenantriebskopf**
Infusion pump with drive device and blockage device for an infusion syringe piston drive head
Pompe à perfusion avec dispositif d'entraînement et dispositif the blocage d'une tête d'entraînement de piston de seringue

(43) Veröffentlichungstag der Anmeldung: 26.06.2013
(73) Patentinhaber: B. Braun Melsungen AG, 34212 Melsungen (DE)
(72) Erfinder: Gerlach, Hans-Josef, 34431 Marsberg (DE); Wildner, René, 34212 Melsungen (DE); Heitmeier, Rolf, 34225 Baunatal (DE)
(74) Vertreter: Winter, Brandl, Fürniss, Hübner, Röss, Kaiser, Polte - Partnerschaft mbB

(56) Entgegenhaltungen:
- EP-A1- 1 329 232
- EP-A2- 1 374 932
- WO-A1-2011/039250
- DE-C1- 4 213 172
- US-A- 5 545 140

## Beschreibung

Die Erfindung betrifft eine Antriebsvorrichtung eines Spritzenpumpenantriebkopfes zum Bewegen eines Infusionsspritzenkolbens umfassend eine Vortriebseinrichtung mit einem Vortriebschlitten, mit einer Vortriebspindel und mit einer wenigstens eine radial bewegbare Mutterschale aufweisenden mehrteiligen Vortriebspindelmutter und umfassend eine mittels eines Kontrollsensors automatisch auslösbare Blockiereinrichtung zum Blockieren einer Zustellbewegung des Antriebkopfes, bei welchem der Vortriebschlitten mittels der Vortriebsspindelmutter an der Vortriebsspindel treibbar ist und bei welchem der Antriebkopf an dem Vortriebschlitten angeordnet ist.

Darüber hinaus betrifft die Erfindung eine Infusionspumpe mit einem Antriebskopf zum Bewegen eines Infusionsspritzenkolbens einer an der Infusionspumpe angeordneten Infusionsspritze, mit einer Vortriebseinrichtung zum Treiben des Antriebskopfes und mit einer Sicherungseinrichtung zum Verhindern einer unbeabsichtigten Bolusgabe.

Auch betrifft die Erfindung ein Verfahren zum Betreiben einer Infusionspumpe, bei welchem ein Antriebskopf für einen Infusionsspritzenkolben einer Infusionsspritze mittels einer Vortriebseinrichtung vorwärts getrieben wird, bei welchem zum Treiben des Antriebskopfes eine mehrteilige Vortriebsspindelmutter entlang einer Vortriebsspindel bewegt wird und bei welchem ein Betätigungselement einer Sicherungsvorrichtung zum Verhindern einer unbeabsichtigten Bolusgabe ausgelöst wird, wenn der Antriebskopf bei einem Infusionsspritzenwechsel bis an den Infusionsspritzenkolben heran bewegt wird.

Insbesondere gattungsgemäße Antriebsvorrichtungen sind aus dem Stand der Technik gut bekannt und werden seit langem in der Infusionstherapie erfolgreich eingesetzt.

Beispielsweise ist aus der WO 2011/039250 A1 eine Methode zum Verhindern einer unbeabsichtigten Bolusgabe hinsichtlich einer händischen Zustellbewegung einer Schiebeeinrichtung für einen Infusionsspritzenkolben insbesondere nach einem Wechsel einer Infusionsspritze an einer Infusionspumpe sowie die entsprechende Infusionspumpe hierfür bekannt. Die Infusionspumpe weist neben der Schiebeeinrichtung zum Schieben des Infusionsspritzenkolbens insbesondere eine Infusionsspritzenaufnahme zum Festlegen einer Infusionsspritze, einen eine zweiteilige Vortriebsspindelmutter umfassende Vortrieb zum Treiben der Schiebeeinrichtung sowie eine Einrichtung zum Blockieren der händischen Zustellbewegung der Schiebeeinrichtung auf. Hierbei zeichnet sich der Vortrieb speziell durch eine Vortriebsspindel und eine Vortriebskupplung zum Kuppeln bzw. Entkuppeln der Schiebeeinrichtung mit bzw. von der Vortriebsspindel aus, wodurch die Schiebeeinrichtung im entkuppelten Zustand der Kupplung vorteilhafter Weise händisch schnell dem Infusionsspritzenkolben der neu eingelegten Infusionsspritze zugestellt werden kann. Dieses schnelle händische Zustellen ist besonders in kritischen Situationen vorteilhaft, wenn es geboten ist, einem Patienten schnell eine wichtige Infusion zuführen zu müssen. Um hierbei jedoch die Gefahr einer nicht ungefährlichen unbeabsichtigten Bolusgabe an dem Patienten reduzieren zu können, verfügt die Infusionspumpe über eine Blockiereinrichtung, mittels welcher die händische Zustellbewegung schnell unterbunden werden kann, wenn die Schiebeeinrichtung dem Infusionsspritzenkolben ausreichend nah händisch zugestellt wurde. Die Blockiereinrichtung zeichnet sich im Speziellen durch eine der Vortriebsspindel nebengeordneten Kontrollgewindewelle mit einer hiermit unlösbar korrespondierenden Gewindewellenmutter und durch eine Bremseinrichtung für die Kontrollgewindewelle aus. Die Gewindewellenmutter ist mit der Schiebeeinrichtung fest verbunden, sodass die händische Zustellbewegung sofort blockiert, sobald die Bremseinrichtung eine Rotation der Kontrollgewindewelle blockt. Die Blockiereinrichtung wird durch eine der Schiebeeinrichtung zugeordneten Signaleinrichtung gesteuert, welche zumindest einen Sensor umfasst, der eine Annäherung der Schiebeeinrichtung an den Infusionsspritzenkolben detektieren kann. Hierdurch kann ein unbeabsichtigtes bzw. kritisches Anstoßen an den Infusionsspritzenkolben im Zuge einer schnellen händischen Zustellbewegung betriebssicher verhindert werden. Zum Blockieren der Kontrollgewindewelle weist die Blockiereinrichtung eine elektromagnetische Bremse auf, welche mit der Signaleinrichtung elektrisch verbunden ist.

Weiterhin offenbart die Druckschrift EP1374932A2 eine Spritzenpumpe mit einem Gehäuse, das ein Bedienfeld an einem aufklappbaren deckel des Gehäuses hat. Zudem weist der Deckel ein Fenster mit einer Lupe auf, durch das ein Benutzer die Spritze sehen kann. Diese Anordnung dient dem Zweck, die Spritzenpumpe in einem hochverdichteten Modulsystem zu miniaturisieren.

Aus der EP1329232A1 ist zudem eine Spritzenpumpe mit einer Aufnahmeposition für eine Spritze bekannt. Zudem weist die Spritzenpumpe einen Antriebskopf auf, der an einer Antriebsstange befestigt ist. Vor einer Fixierung der Kolbenplatte einer Spritze im Antriebskopf wird die Kolbenstange der Spritze mit einem Bremselement verriegelt. Nach dem Ansetzen des Spritzenbügels gegen einen Spritzenzylinder wird das Bremselement betätigt, um an der Kolbenstange anzugreifen und diese in Bezug auf den Spritzenzylinder festzuhalten.

Eine weitere Spritzenpumpe, welche eine Mehrzahl an verschieden großen Spritzen aufnehmen kann, ist aus der Druckschrift US5545140 bekannt. Weiterhin weist die Spritzenpumpe einen Detektor auf, der eine Warnung ausgibt, wenn, wenn eine Spritze nicht richtig in die Spritzenpumpe eingebracht ist.

Zudem ist aus der Druckschrift DE 4213172 C1 ein Drucksinfusionsapparat bekannt, der einen Kolbenbremse ausweist, die die Bewegung eines Spritzenkolbens blockiert, solange die Spritze nicht korrekt in dem Druckinfusionsapparate positioniert ist. Somit werden unbeabsichtigte Verschiebungen des Spritzenkolbens durch Saugwirkung oder mechanisches Bewegen vor Infusionsbeginn vermieden.Es ist Aufgabe vorliegender Erfindung eine unbeabsichtigte Bolusgabe an einen Patienten insbesondere im Zuge eines Infusionsspritzenwechsels konstruktiv anders zu gestalten.

Die Aufgabe der Erfindung wird von einer Antriebsvorrichtung eines Infusionsspritzenkolbenantriebskopfes zum Bewegen eines Infusionsspritzenkolbens umfassend eine Vortriebseinrichtung mit einem Vortriebsschlitten, mit einer Vortriebsspindel und mit einer
wenigstens eine radial bewegbaren Mutterschale aufweisenden mehrteiligen Vortriebsspindelmutter und umfassend eine Blockiereinrichtung zum Blockieren einer Zustellbewegung des Antriebskopfes gelöst, bei welchem der Vortriebsschlitten mittels der Vortriebsspindelmutter an der Vortriebsspindel treibbar ist und bei welchem der Antriebskopf an dem Vortriebsschlitten angeordnet ist, bei welchem erfindungsgemäß die mittels eines Kontrollsensors automatisch auslösbare Blockiereinrichtung die mehrteilige Vortriebsspindelmutter zum Blockieren der Zustellbewegung des Antriebskopfes an der Vortriebsspindel umfasst.

Dadurch, dass die mittels eines Kontrollsensors automatisch auslösbare Blockiereinrichtung direkt die mehrteilige Vortriebsspindelmutter umfasst, kann die gesamte Antriebsvorrichtung des Antriebskopfes konstruktiv wesentlich einfacher gebaut werden, sodass speziell die händische Zustellbewegung an der Vortriebsspindel baulich extrem einfach blockierbar ist. Insbesondere kann im Hinblick auf den eingangs erläuterten Stand der Technik auf eine zweite Gewindewelle mitsamt der elektromagnetischen Bremse verzichtet werden, wodurch die vorliegende Antriebsvorrichtung wesentlich kompakter gebaut werden kann.

Insofern gestaltet sich ein Einlegen einer neuen Infusionsspritze in eine Infusionspumpe wesentlich einfacher und ein Wechsel von Infusionsspritzen kann damit auch schneller durchgeführt werden. Und dennoch kann die Gefahr einer Bolusgabe für einen Patienten zuverlässig verhindert werden.

Der Begriff "Mutterschale" beschreibt im Sinne der Erfindung einen Schalenteil einer in ihrer Längserstreckung längsgeschnittenen Vortriebsspindelmutter, wobei das Schalenteil von der Vortriebsspindel radial an- bzw. abgehoben werden kann. Vorzugsweise umfasst die Vortriebsspindelmutter wenigstens zwei derartige Mutterschalen, sodass ein gleichmäßiger Eingriffskontakt zwischen der Vortriebsspindel und der Vortriebsspindelmutter gewährleistet werden kann. Hierdurch kann die Arbeitspräzision nochmals erhöht werden.

Idealerweise ist die Vortriebsspindelmutter an dem bzw. in dem Vortriebsschlitten gelagert bzw. angeordnet, sodass eine robuste und kompakt bauende Einheit an der Vortriebseinrichtung bereitgestellt werden kann.

Bevorzugt weist die Vortriebsspindelmutter der Blockiereinrichtung wenigstens zwei Mutterschalen auf, wobei die wenigstens zwei Mutterschalen jeweils ein mit einem Außengewinde der Vortriebsspindel korrespondierbares Innengewinde aufweisen, wodurch die Vortriebsspindelmutter symmetrisch mit der Vortriebsspindel korrespondieren kann.

Insofern wird die Aufgabe der Erfindung auch von einer Infusionspumpe mit einem Antriebskopf zum Bewegen eines Infusionsspritzenkolbens einer an der Infusionspumpe angeordneten Infusionsspritze, mit einer Vortriebseinrichtung zum Treiben des Antriebskopfes und mit einer Sicherungsvorrichtung zum Verhindern einer unbeabsichtigten Bolusgabe gelöst, wobei sich die Infusionspumpe durch eine Antriebsvorrichtung nach einem der hier beschriebenen Merkmale auszeichnet.

Vorteilhafter Weise erhöht sich aufgrund des einfacheren Aufbaus auch die Betriebssicherheit der Infusionspumpe, da weniger Bauteile vorhanden sind, und hierdurch speziell die Ausfallwahrscheinlichkeit reduziert werden kann. Insofern kann trotz einer schnelleren Wechselmöglichkeit einer Infusionsspritze an einer Infusionspumpe die Gefahr einer unkontrollierten Infusion weiter signifikant reduziert werden.

Bei der vorliegenden Infusionspumpe handelt es sich insbesondere um eine Spritzenpumpe, in welcher wenigstens eine Infusionsspritze eingelegt werden kann. Die vorliegende Infusionspumpe kann sowohl als Einzelpumpe als auch in einem Ordnungssystem bzw. einer Docking-Station zusammengefasst betrieben werden.

Es versteht sich, dass die Blockiereinrichtung konstruktiv unterschiedlich realisiert werden kann. Beispielsweise können unterschiedlichste Axialschiebeeinrichtungen hinsichtlich der Blockiereinrichtung vorgesehen sein. Eine bevorzugte Ausführungsvariante sieht jedoch vor, dass die Blockiereinrichtung eine axial verschiebliche Auslösehülse umfasst, welche die Vortriebspindelmutter radial außen umgibt, wodurch die Mechanik radial besonders schlank baut.

Es ist vorteilhaft, wenn die Blockiereinrichtung Mittel zum Beschleunigen einer Auslösehülse umfasst. Mittels der Beschleunigungsmittel kann eine geringe Reaktionszeit der Blockiereinrichtung erreicht werden.

Weisen die Beschleunigungsmittel insbesondere eine Beschleunigungsspiralfeder auf, welche radial außen auf der Auslösehülse angeordnet ist, können die Beschleunigungsmittel mechanisch einfach umgesetzt werden. Auch hierdurch kann eine platzsparende Mechanik bereitgestellt werden.

In diesem Zusammenhang ist es vorteilhaft, wenn die wenigstens eine radial bewegbare Mutterschale ein in Bezug auf ihre Längserstreckung axial verschiebliches Betätigungselement aufweist, welches in Bezug auf die Vortriebsspindel ein radiales Betätigen der wenigstens einen radial bewegbaren Mutterschale bewirkt.

Nach einem weiteren Aspekt der Erfindung wird die vorliegende Aufgabe auch von einem Verfahren zum Betreiben einer Infusionspumpe gelöst, bei welchem ein Antriebskopf für einen Infusionsspritzenkolben einer Infusionsspritze mittels einer Vortriebseinrichtung vorwärts getrieben wird, bei welchem zum Treiben des Antriebskopfes eine mehrteilige Vortriebsspindelmutter entlang einer Vortriebsspindel bewegt wird und bei welchem ein Betätigungselement einer Sicherungsvorrichtung zum Verhindern einer unbeabsichtigten Bolusgabe ausgelöst wird, wenn der Antriebskopf bei einem Infusionsspritzenwechsel bis an den Infusionsspritzenkolben heran bewegt wird, wobei das Betätigungselement erfindungsgemäß axial entlang der Vortriebsspindel verschoben wird, wodurch die mehrteilige Vortriebsspindelmutter zumindest teilweise radial auf die Vortriebsspindel zubewegt oder radial von der Vortriebsspindel fortbewegt wird.

Durch diese Bewegungstransformation von der axial gerichteten Bewegung des Betätigungselements in die radial gerichtete Bewegung der mehrteiligen Vortriebsspindelmutter oder Teile hiervon, kann eine außergewöhnlich effektiv arbeitende Blockiereinrichtung der Sicherheitsvorrichtung realisiert werden.

Vorzugsweise umfasst das Betätigungselement jedoch axial betätigbare Schiebehülsen, mittels welchen insbesondere ein radiales Zupacken der Vortriebsspindelmutter an der Vortriebsspindel ermöglicht wird. Schiebehülsen haben den Vorteil, dass sie die Vortriebsspindelmutter sowohl radial als auch axial zumindest teilweise sehr großzügig umschließen können, wodurch eine besonders gute Führung der Schiebehülsen insbesondere gegenüber einer oder mehrerer Mutterschalen der Vortriebsspindelmutter erzielbar ist. Anstelle der Schiebehülsen können jedoch auch andere Axialschiebeeinrichtungen vorgesehen sein. Beispielsweise kann eine derartige Axialschiebeeinrichtung einen Schiebering, einen Schiebekäfig oder einen sonstig gestalteten gegebenenfalls auch linear ausgebildeten Axialschieber umfassen.

Des Weiteren ist es vorteilhaft, wenn die wenigstens eine radial bewegbare Mutterschale radial außen von wenigstens einer Schiebehülse, vorzugsweise von zwei Schiebehülsen, zumindest teilweise umgeben ist, wobei die wenigstens eine radial bewegbare Mutterschale von wenigstens einer Schiebehülse, vorzugsweise von zwei Schiebehülsen, radial verlagerbar ist. Hierdurch kann um die Vortriebsspindel herum eine mechanisch besonders einfach bauende Betätigung bereitgestellt werden.

An dieser Stelle sei erwähnt, dass die Antriebsvorrichtung äußerst vorteilhaft weiterentwickelt werden kann, wenn die Blockiereinrichtung mittels einer Schiebehülse oder vorzugsweise wenigstens zwei Schiebhülsen steuerbar ist, wobei die wenigstens zwei Schiebehülsen einzeln antreibbar und/oder sequentiell verschiebbar angeordnet sind. Hierdurch kann das Ansprechverhalten der Blockiereinrichtung wesentlich verbessert werden.

Hierbei spielt es keine vordergründige Rollen, ob es sich bei den wenigstens zwei Schiebehülsen um zwei Einzelschiebehülsen oder um eine in Axialrichtung in zwei Teilschiebehülsen geteilte mehrteilige Schiebehülse handelt, solange die Teilschiebehülsen einzeln antreibbar und sequentiell verschiebbar sind und die Blockiereinrichtung hierdurch im Sinne der Erfindung steuerbar ist.

Die Zuverlässigkeit der vorliegenden Blockiereinrichtung kann wesentlich verbessert werden, wenn die wenigstens eine radial bewegbare Mutterschale einen Eingreifbereich zum Eingreifen in ein Außengewinde der Vortriebsspindel und jeweils einen Auflagebereich zum radialen Aufliegen auf das Außengewinde der Vortriebspindel aufweist, wobei der Eingreifbereich und der Auflagebereich axial hintereinander angeordnet sind. Je nach konkreter Ausgestaltung kann es jedoch auch ausreichend sein, wenn nur eine einzige Mutterschale einen derartigen Eingriffsbereich aufweist.

Insbesondere ein Öffnen der Vortriebsspindelmutter kann konstruktiv einfach bewerkstelligt werden, wenn die wenigstens eine radial bewegbare Mutterschale endseitig auf einem Vortriebsspindellager gelagert ist.

Vorteilhafter Weise ist die wenigstens eine radial bewegbare Mutterschale endseitig auf einem Vortriebsschlittenlager gelagert, sodass die Mutterschale an diesem Lagerbereich besonders einfach schwenkbar gelagert und hierbei der Eingreifbereich der Mutterschale von dem Außengewinde insbesondere entkoppelt der werden kann.

Hierzu weist die Mutterschale vorteilhafter Weise jeweils einen Drehlagerbereich auf, der idealerweise endseitig an jeder der Mutterschalen angeordnet ist.

Vorzugweise befindet sich ein derartiger Drehlagerbereich an einem dem Eingriffsbereich abgewandten Ende der jeweiligen Mutterschale, sodass ein ausreichend langer Hebelarm zwischen dem Eingriffsbereich und dem Drehlagerbereich zur Verfügung gestellt werden kann, um ein vorteilhaftes Koppeln und Entkoppeln der Vortriebsspindelmutter erzielen zu können.

Eine konstruktiv weiter vereinfachte Ausführungsvariante sieht vor, dass die wenigstens eine radial bewegbare Mutterschale im Wesentlichen quer zu ihrer Längserstreckung kippbar gegenüber der Vortriebsspindel angeordnet ist. Hierdurch kann ein radiales Öffnen und Schließen der Vortriebsspindelmutter konstruktiv besonders einfach möglich werden, sodass speziell auch auf radial wirkende Federeinrichtungen verzichtet werden kann.

Das Betätigungselement kann vorliegend besonders gut integriert werden, wenn die Vortriebspindelmutter einen Kopfbereich und einen Fußbereich aufweist, deren jeweilige Außendurchmesser größer sind als ein zwischen dem Kopf- und Fußbereich angeordnetes Vortriebsspindelmuttergebiet.

Ein radiales Betätigen der Vortriebsspindelmutter kann vorteilhafter gestaltet werden, wenn der Kopfbereich eine Rampe für axial verschiebliche Schiebehülsen und der Fußbereich ein Gegenlager für wenigstens eine der axial verschieblichen Schiebehülsen ausgestaltet. Beispielsweise kann die mehrteilige Vortriebsspindelmutter vorteilhaft geschlossen bzw. in Eingriff mit dem Außengewinde der Vortriebsspindel gebracht werden, wenn wenigstens eine der Schiebehülsen axial über die Rampe der Vortriebsspindelmutter geschoben und hierbei wenigstens eine Mutterschale radial nach innen und auf die Vortriebsspindel zu bewegt wird.

Eine besonders bevorzugte Ausführungsvariante sieht vor, dass eine axial verschiebliche Auslösehülse und eine axial verschiebliche Freigabehülse jeweils eine Schließposition und eine Öffenposition aufweisen.

Mittels der Auslösehülse kann die Blockiereinrichtung insbesondere so blockiert werden, dass die Vortriebsspindelmutter schlagartig mit der Vortriebsspindel formschlüssig verbunden wird, wenn der Antriebskopf mit einem Infusionsspritzkolben in Wechselwirkung tritt.

Mittels der Freigabehülse kann insbesondere die Gewindeverbindung zwischen der Vortriebsspindel und der Vortriebsspindelmutter gelöst werden, wenn die Antriebsvorrichtung händisch bedient werden soll.

Weist die Vortriebseinrichtung eine Parkeinrichtung zum zwischenzeitlichen Parken einer Schiebehülse auf, welche axial neben der Vortriebsspindelmutter angeordnet ist, kann die Schiebehülse vorteilhaft in eine Öffenposition hinein verlagert werden.

Darüber hinaus ist es vorteilhaft, wenn die Vortriebseinrichtung steuerbare Elektrohaltemagneten zum positionierten Halten von Schiebehülsen der Vortriebspindelmutter umfasst.

Beispielweise kann wenigstens eine der Schiebehülsen gegen die Vorspannkraft einer Vorspannfeder in einer Öffenposition gehalten werden, sodass sie beim Deaktivieren des entsprechenden Elektrohaltemagneten in eine Schließposition hinein schnellen kann.

Vorzugweise sind die Elektrohaltemagnete elektrisch bzw. elektronisch steuerbar.

Dementsprechend sieht eine vorteilhafte Ausführungsvariante vor, dass die Vortriebseinrichtung insbesondere zum Halten der Vortriebsspindelmutter in einer geöffneten Betriebsstellung steuerbare Elektrohaltemagnete umfasst.

Kumulativ oder alternativ können derartig steuerbare Elektromagnete auch zum Halten der Vortriebsspindelmutter in einer geschlossenen Stellung vorhanden sein, beispielsweise um eine Schiebehülse temporär in einer Schließposition zu halten.

Vorteilhafter Weise sind die steuerbaren Elektrohaltemagnete zum Halten der vorhandenen Schiebehülsen in ihren jeweiligen Öffenpositionen an dem Vortriebsschlitten angeordnet bzw. in diesem integriert.

Auch ist es vorteilhaft, wenn die Antriebsvorrichtung eine Einrichtung zum manuellen Positionieren eines Betätigungselements der wenigstens einen Mutterschale aufweist.

Beispielsweise kann eine Schiebehülse durch manuelles Umlegen eines Bedienhebels in Richtung eines Elektromagneten umpositioniert werden, während gleichzeitig der Elektromagnet durch das Bedienen des Bedienhebels aktiviert wird.

Vorteilhafter Weise kann die Positioniereinrichtung eine Positionierungswelle zum Positionieren von Schiebehülsen der Vortriebspindelmutter aufweisen, sodass ein manuelles Betätigen insbesondere der Vortriebsspindelmutter konstruktiv problemlos über die Einrichtung zum manuellen Positionieren möglich ist, die etwa an der Rückseite des Antriebskopfes entfernt von der Vortriebseinrichtung angeordnet ist.

An dieser Stelle sei nochmals erwähnt, dass der Antriebskopf für eine Kolbenplatte der Infusionsspritze ein Gegenlager zur Vortriebseinrichtung bietet. Die Kolbenplatte der Infusionsspritze findet hier ihre Abstützung. Durch eine innere Bewegung der Vortriebseinrichtung wird die Infusionsspritze linear ausgedrückt. Am Antriebskopf wird die Kolbenplatte der Infusionsspritze mittels zweier federbelasteter Fixierbügel einer Haltekralle sicher fixiert. Weiterhin halten die Fixierbügel die Kolbenplatte selbst verschiedenster Infusionsspritzen form- und kraftschlüssig an einem Membranteller des Antriebskopfes, wodurch ein zeitnahes Anlaufverhalten, sowie eine axiale Fixierung des Spritzenkolbens auch bei einem anstehenden Unterdruck in der Infusionsspritze gewährleistet werden kann. Der Membranteller bewegt sich durch Kontakt mit der Kolbenplatte in den Antriebskopf hinein, wobei nacheinander zwei Lichtschranken einer Zwei-Schaltschwellen-Einrichtung durchfahren werden. Mittels der beiden Lichtschranken werden der Formschluss zwischen der Vortriebsspindelmutter und der Vortriebsspindel sowie das spielfreie Heranführen des Antriebskopfes an die Kolbenplatte gesteuert, ohne dass hierbei eine Bolusgabe erzeugt wird. Weiterhin befindet sich im Antriebskopf ein Kraftsensor zur Ermittlung der anstehenden Antriebskraft. Mit Betätigung des rückseitig an dem Antriebskopf angeordneten Bedienhebels werden die Haltekralle geöffnet und der Formschluss zwischen der Vortriebsspindelmutter und der Vortriebsspindel aufgehoben. Die Bedienung des Bedienhebels wird vorzugsweise mittels entsprechender Mikroschalter erkannt und die Elektrohaltemagnete hierdurch gesteuert. Das Öffnen der Haltekralle bewirkt zudem ein Herausbewegen des federbelasteten Membrantellers aus dem Antriebskopf heraus.

Vorteilhafter Weise wird mittels der vorliegenden Erfindung eine besonders gute automatische Infusionsspritzenfixierung hinsichtlich einer Infusionspumpe erzielt. Hierbei zeichnet sich die Erfindung insbesondere durch die Möglichkeit aus, dass der Antriebskopf besonders schnell an die Kolbenplatte heran bewegt werden kann. Die Zeitdauer eines Infusionsspritzenwechsels kann hierbei noch besser von dem Anwender bestimmt werden, was besonders bei kritischen Behandlungssituationen von Vorteil ist. Trotzdem kann hierbei besonders betriebssicher gewährleistet werden, dass ein nahezu bolusfreies Einlegen der Infusionsspritze sowie ein zeitnahes Anlaufverhalten ermöglicht werden.

Weitere Vorteile, Ziele und Eigenschaften vorliegender Erfindung werden anhand anliegender Zeichnung und nachfolgender Beschreibung erläutert, in welchen beispielhaft eine Infusionspumpe mit eine erfindungsgemäße Antriebsvorrichtung dargestellt und beschrieben ist. In der Zeichnung zeigen:
- Figur 1: schematisch eine Ansicht einer Infusionspumpe mit einer daran angeordneten Infusionsspritze;
- Figur 2: schematisch eine Ansicht einer Antriebsvorrichtung der Infusionspumpe aus der Figur 1 mit einem Infusionsspritzenkolbenantriebskopf und mit einer Vortriebseinrichtung hierfür;
- Figur 3A: schematisch eine Detailansicht der Vortriebseinrichtung aus der Figur 2 in einer beispielhaft ausgewählten ersten Betriebsstellung;
- Figur 3B: schematisch eine Ansicht eines Längsschnitts der Detailansicht aus der Figur 3A;
- Figur 4A: schematisch eine weitere Detailansicht der Vortriebseinrichtung aus den Figuren 2 und 3 in einer beispielhaft ausgewählten zweiten Betriebsstellung;
- Figur 4B: schematisch eine Ansicht eines Längsschnitts der Detailansicht aus der Figur 4A;
- Figur 5A: schematisch eine zusätzliche Detailansicht der Vortriebseinrichtung aus den Figuren 3 und 4 in einer beispielhaft ausgewählten dritten Betriebsstellung;
- Figur 5B: schematisch eine Ansicht eines Längsschnitts der Detailansicht aus der Figur 5A; und
- Figur 6: schematisch eine Ansicht eines Prozessablaufes zur Verhinderung einer unbeabsichtigten Bolusgabe an einen Patienten.

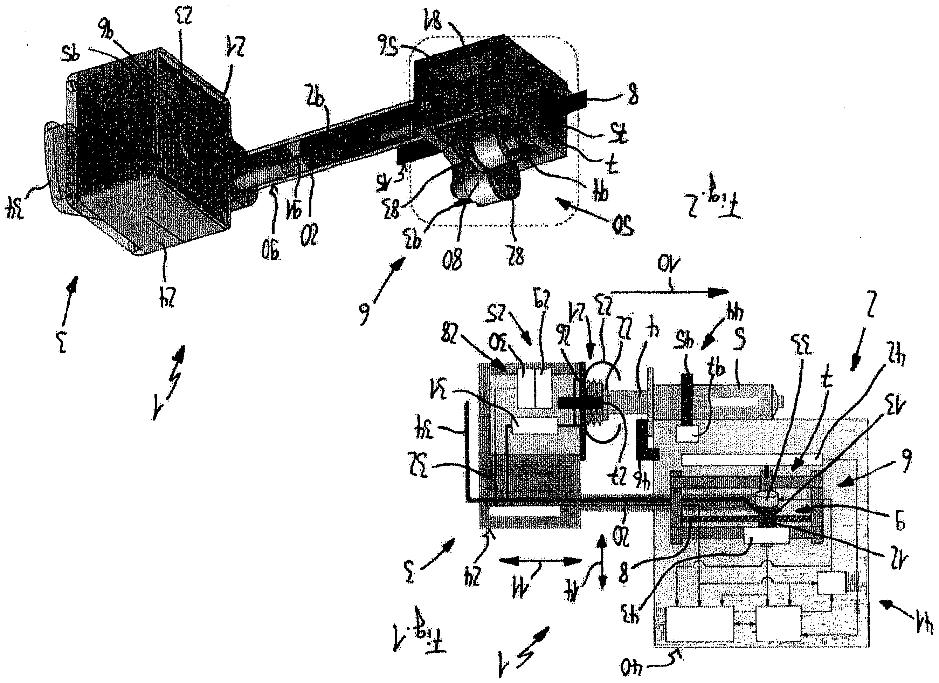

Die in den Figuren 1 bis 5 gezeigte Antriebsvorrichtung 1 einer Infusionspumpe 2 umfasst im Wesentlichen einen Antriebskopf 3 zum Bewegen eines Kolbens 4 einer an der Infusionspumpe 2 befestigten Infusionsspritze 5 und eine Vortriebseinrichtung 6 für den Antriebskopf 3.

Die Vortriebseinrichtung 6 umfasst im Wesentlichen einen Vortriebsschlitten 7, eine Vortriebsspindel 8 sowie eine mehrteilige Vortriebsspindelmutter 9. Darüber hinaus umfasst er noch einen Vortriebsmotor und ein entsprechendes Getriebe, welche vorliegend jedoch nicht dargestellt sind. Mit der Vortriebseinrichtung 6 wird eine lineare Zustellbewegung des Antriebskopfes 3 umgesetzt.

Im Vortriebsschlitten 7 sind die lineare Führung und der Verdrehschutz des Antriebskopfes 3 gewährleistet.

Die mehrteilige Vortriebsspindelmutter 9 bzw. der Vortriebsschlitten 7 können bei entsprechend gewählter Drehrichtung der Vortriebsspindel 8 in Kolbenantriebsrichtung 10 vorwärts angetrieben werden, wobei insbesondere die mehrteilige Vortriebsspindelmutter 9 axial entlang der Längserstreckung 11 der Vortriebsspindel 8 bewegt wird.

Die mehrteilige Vortriebsspindelmutter 9 umfasst hierbei eine erste radial bewegbare Mutterschale 12 und eine zweite radial bewegbare Mutterschale 13, wie später noch ausführlicher beschrieben ist. Jedenfalls können die radial bewegbaren Mutterschalen 12 und 13 einen axialen Formschluss mit der Vortriebsspindel 8 gewährleisten und diesen jedoch auch aufheben.

Der Begriff "radial" kennzeichnet eine radiale Bewegungsrichtung 14 im Wesentlichen quer zur Längserstreckung 11 der Vortriebsspindel 8. Die Vortriebsspindel 8 weist ein Außengewinde 15 auf, mittels welchem die mehrteilige Vortriebsspindelmutter 9 formschlüssig verbunden werden kann.

Der Antriebskopf 3 ist mittels eines Haltearms 20 an dem Vortriebsschlitten 7 befestigt. Der Antriebskopf 3 umfasst einen Membranteller 21, an welchem eine Kolbenplatte 22 des Infusionsspritzenkolbens 4 abgestützt werden kann. Hierbei wird die Kolbenplatte 22 mittels Haltekralle 23 an dem Antriebskopf 3 fixiert, sodass eine formschlüssige Verbindung zwischen dem Infusionsspritzenkolben 4 und dem Antriebskopf 3 garantiert ist.

Der Antriebskopf 3 beherbergt in seinem Antriebskopfgehäuse 24 einen Kolbenplattensensor 25, der zum einen ein Feder 26 vorgespanntes axial verlagerbares Geberelement 27 und zum anderen eine durch das Geberelement 27 beeinflussbare Zwei-Schaltschwellen-Einrichtung 28 mit einer ersten Lichtschranke 29 und einer zweiten Lichtschranke 30 umfasst.

Mittels des Kolbenplattensensors 25 kann einerseits die Funktion eines Antriebsgetriebes 31 der Haltekralle 23 gesteuert werden. Andererseits kann mittels des Kolbenplattensensors 25 die Funktion der Vortriebseinrichtung 6 beeinflusst werden, wie später noch detaillierter beschrieben ist. Hierzu ist der Kolbenplattensensor 25 über eine entsprechende Signalleitung 32 mit einer Steuerung 33 der Vortriebseinrichtung 6 verbunden.

An dem Antriebskopf 3 ist noch ein manuell bedienbarer Bedienhebel 34 vorgesehen, mittels welchen die Funktionen der Haltekralle 23 und der Vortriebseinrichtung 6 zusätzlich manuell betätigt werden können. Zur mechanischen Betätigung der Vortriebseinrichtung 6 mittels des Bedienhebels 34 ist dieser von dem Antriebskopf 3 durch den Haltearm 20 hindurch bis zur Vortriebseinrichtung 6 geführt.

Die speziell in der Figur 1 schematisch illustrierte Infusionspumpe 2 weist ein Infusionspumpengehäuse 40 auf, in welchem neben der Vortriebseinrichtung 6 auch weitere Funktionsbauteile, wie etwa weitere Regel- und/oder Steuereinrichtungen 41 untergebracht sind, auf die hier jedoch nur insoweit eingegangen sind, sofern sie für die vorliegende Erfindung wesentlich sind. So umfassen die Regel- und/oder Steuereinrichtungen 41 etwa einen Sensor 42 zum Detektieren der korrekten Einbaulage der Infusionsspritze 5 und etwa einen Formschlusssensor 43 zum Kontrollieren eines Formschlusszustandes zwischen der mehrteiligen Vortriebsspindelmutter 9 und der Vortriebsspindel 8. An dem Infusionspumpengehäuse 40 von außen zugänglich ist noch eine Aufnahme 44 für die Infusionsspritze 5 vorgesehen, welche einen Klemmbügel 45 zum radialen Festlegen der Infusionsspritze 5 und eine Klemmlasche 46 zum axialen Festlegen der Infusionsspritze 5 umfasst.

Vorteilhafter Weise umfasst die Infusionspumpe 2 noch eine Sicherungsvorrichtung 50 zum Verhindern einer unbeabsichtigten Bolusgabe an einem Patienten insbesondere bei einem Infusionsspritzenwechsel, bei welchem eine gebrauchte Infusionsspritze 5 gegen eine neue Infusionsspritze 5 ausgewechselt werden muss (siehe insbesondere Figur 2).

Die Sicherungsvorrichtung 50 umfasst wiederum eine mittels des Kolbenplattensensors 25 automatisch auslösbare Blockiereinrichtung 51 zum Blockieren einer händischen Zustellbewegung des Antriebskopfes 3 in Kolbenantriebsrichtung 10, die erfindungsgemäß direkt die mehrteilige Vortriebsspindelmutter 9 umfasst.

Die Blockiereinrichtung 51 umfasst vorteilhafter Weise ein axial verschiebliches Betätigungselement 52, welches hier beispielhaft zumindest eine axial verschiebliche Auslösehülse 53 umfasst, die konzentrisch um die beiden Mutterschalen 12 und 13 der Vortriebsspindelmutter 9 herum angeordnet ist. Die Auslösehülse 53 ist hierbei in Längserstreckung 11 der Vortriebsspindel 8 verschieblich radial außen an der Vortriebsspindelmutter 9 gelagert.

Damit die Blockiereinrichtung 51 extrem schnell aktiviert werden kann, verfügt sie über Mittel 54 zum Beschleunigen der Auslösehülse 53, wobei die Beschleunigungsmittel 54 konstruktiv einfach durch eine Spiralfeder 55 ausgestaltet sind, die wiederum radial außen an der Auslösehülse 53 gelagert ist.

Darüber hinaus verfügt das Betätigungselement 52 noch über eine Freigabehülse 56, die ebenfalls axial verschieblich und konzentrisch um die beiden Mutterschalen 12 und 13 der Vortriebsspindelmutter 9 herum angeordnet sein kann.

Insofern sind beide Schiebehülsen 53 und 56 auch axial in Bezug auf die Längserstreckung 57 der beiden radial bewegbaren Mutterschalen 12 und 13 verschieblich gelagert. Die beiden Schiebehülsen 53, 56 sind hierbei einzeln angetrieben und sequentiell verschiebbar, wie nachfolgend auch noch ausführlicher beschrieben ist.

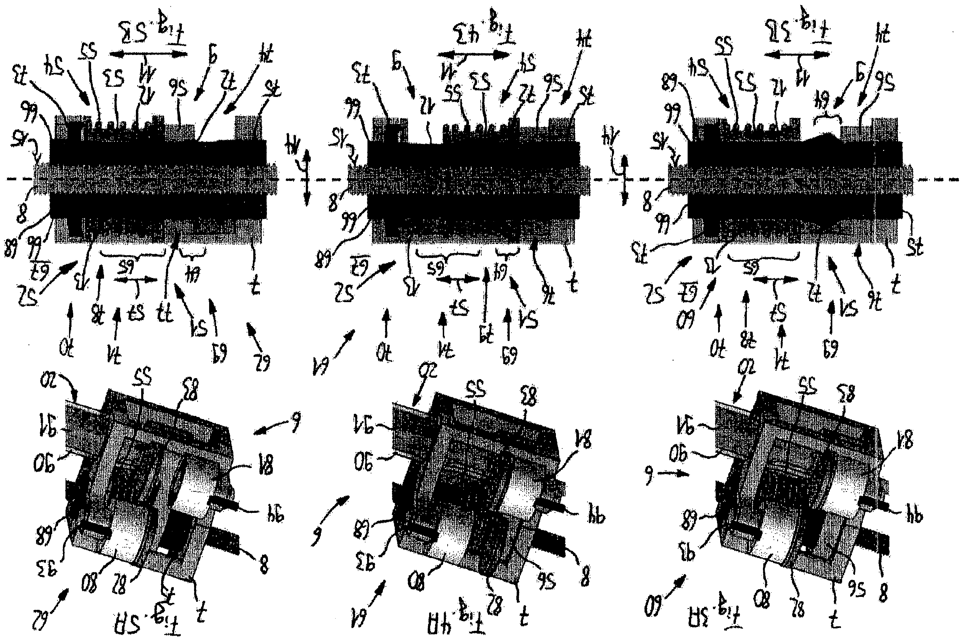

Zum besseren Verständnis der Funktionsweise des vorliegenden Betätigungselements 52 der Vortriebsspindelmutter 9 ist in den Figuren 3A und 3B beispielhaft eine erste Betriebsstellung 60 der Vortriebseinrichtung 6 gezeigt, bei welcher die beiden Mutterschalen 12 und 13 sich nicht im formschlüssigen Eingriff mit dem Außengewinde 15 der Vortriebsspindel 8 befinden. In dieser ersten Betriebsstellung 60 kann der Antriebskopf 3 mittels einer händischen Zustellbewegung in Kolbenantriebsrichtung 10 noch schnell an die Kolbenplatte 22 heran bewegt werden. Diese Betriebsstellung 60 kann insbesondere dann gewählt werden, wenn ein Infusionsspritzenwechsel an der Infusionspumpe 2 vorgenommen wird.

Bei einer in den Figuren 4A und 4B beispielhaft gezeigten zweiten Betriebsstellung 61 der Vortriebseinrichtung 6 ist die Blockiereinrichtung 51 ausgelöst und die Vortriebseinrichtung 6 ist durch die Vortriebsspindelmutter 9 an sich blockiert, sodass eine händische Zustellbewegung des Antriebskopfes 3 in Kolbenantriebsrichtung 10 weiter nicht möglich ist. Insofern wird hierdurch die Gefahr ausgeschlossen, dass der Membranteller 21 kritisch an die Kolbenplatte 22 anstößt und hierdurch versehentlich eine für einen Patienten womöglich gefährliche Bolusgabe erfolgt.

Letztlich befindet sich bei einer weiteren hinsichtlich der Figuren 5A und 5B beispielhaft ausgewählten dritten Betriebsstellung 62 die Vortriebseinrichtung 6 in einem Grundbetrieb, bei welchem der Antriebskopf 3 durch die Vortriebseinrichtung 6 sukzessive in Kolbenantriebsrichtung 10 angetrieben wird und bei welchem somit eine gewünschte Infusionstherapie in an sich bekannter Weise mittels der Infusionspumpe 2 erfolgen kann.

Die beiden radial bewegbaren Mutterschalen 12 und 13 weisen jeweils einen Eingreifbereich 64 zum Eingreifen in das Außengewinde 14 der Vortriebsspindel 8 auf. Insofern kann eine Formschlussverbindung zwischen den Mutterschalen 12 und 13 der Vortriebsspindelmutter 9 und dem Außengewinde 15 der Vortriebsspindel 8 über dem jeweiligen Eingreifbereich 64 der Mutterschalen 12 bzw. 13 hergestellt werden, wenn dieser Eingreifbereich 64 ordnungsgemäß radial in Richtung der Vortriebsspindel 8 bewegt wird bzw. gehalten wird. Dies ist beispielsweise bei den beiden Betriebsstellungen 61 und 62 der Fall. Darüber hinaus weisen die beiden radial bewegbaren Mutterschalen 12 und 13 noch einen Auflagebereich 65 auf, mittels welchen die Mutterschalen 12 und 13 höchstens radial auf das Außengewinde 15 aufliegen können, jedoch nicht mit diesem in Eingriff gelangen können. Der Eingreifbereich 64 und der Auflagebereich 65 sind axial hintereinander angeordnet.

Um die beiden radial bewegbaren Mutterschalen 12, 13 jeweils mit den beiden Schiebehülsen 53, 56 gegenüber der Vortriebsspindel 8 um eine Kippachse 66, welche im Wesentlichen quer zu Längserstreckung 57 der jeweiligen Mutterschale 12 bzw. 13 verläuft, herum radial anstellen zu können, sind die beiden Mutterschalen 12 und 13 endseitig 67 jeweils auf einem Vortriebsschlittenlager 68 gelagert. Die Kippachse 66 erstreckt sich nach der Darstellung der Figuren 3B, 4B und 5B senkrecht zur Papierebene.

Darüber hinaus weist die Vortriebsspindelmutter 9 einen Kopfbereich 69 und einen Fußbereich 70 auf, deren jeweilige Außendurchmesser größer sind als ein dazwischen angeordnetes Vortriebsspindelmuttergebiet 71, wobei der Kopfbereich 69 eine Rampe 72 zum Aufschieben für die beiden axial verschieblichen Schiebehülsen 53 und 56 und der Fußbereich 70 ein Gegenlager 73 für die beiden axial verschieblichen Schiebehülsen 53 und 56 ausgestaltet.

An einem dem Vortriebsschlittenlager 68 gegenüberliegenden Vortriebsspindelmutterende 74 befindet sich eine an dem Vortriebsschlitten 7 gelagerte Parkeinrichtung 75 für die Freigabehülse 56, wobei die Parkeinrichtung 75 axial neben der Vortriebsspindelmutter 9 platziert ist. Ist die Freigabehülse 56 auf den Sitz der Parkeinrichtung 75 aufgeschoben und hierbei neben der Vortriebsspindelmutter 9 geparkt, befindet sich die Freigabehülse 56 in einer Öffenposition 76. Dies ist bei den beiden Betriebsstellungen 60 und 61 der Fall. Bei der dritten Betriebsstellung 62 ist die Freigabehülse 56 auf die Rampe 72 verschoben und sie befindet sich dementsprechend in einer Schließposition 77.

Die Auslösehülse 53 befindet sich in den beiden Betriebsstellungen 60 und 62 jeweils in ihrer eigenen Öffenposition 78, in welcher die Auslösehülse 53 dem Gegenlager 73 näher als der Rampe 72 zugewandt ist. Lediglich bei der Betriebsstellung 61 befindet sich die Auslösehülse 53 in einer ihr zugeteilten Schließposition 79.

Des Weiteren umfasst die Vortriebseinrichtung 8 ein erstes Elektrohaltemagnet 80 und ein zweites Elektrohaltemagnet 81, welche in diesem Ausführungsbeispiel oberhalb des Vortriebsschlittens 7 angeordnet sind.

Der erste Elektrohaltemagnet 80 ist hierbei der Auslösehülse 53 zugeordnet und er kann dementsprechend mit einem Magnetteller 82 der Auslösehülse 53 korrespondieren. Hierzu ist der Magnetteller 82 vor dem ersten Elektrohaltemagnet 80 platziert und gekoppelt mit der Auslösehülse 53 axial verschieblich gelagert. Bei den beiden Betriebsstellungen 60 und 62 ist der erste Elektrohaltemagnet 80 bestromt, sodass er mit dem Magnetteller 82 Kontakt halten und damit auch die Auslösehülse 53 in ihrer Öffenposition 78 halten kann. Bei der Betriebsstellung 61 ist der Magnetteller 82 von dem ersten unbestromten Elektrohaltemagneten 80 beabstandet.

Der zweite Elektrohaltemagnet 81 ist dementsprechend der Freigabehülse 56 zugeordnet und er kann mit einem Magnetteller 83 der Freigabehülse 56 korrespondieren, wobei dieser Magnetteller 83 hierzu vor dem zweiten Elektrohaltemagnet 81 platziert ist. Der Magnetteller 83 wird gemeinsam mit der Freigabehülse 56 axial verschoben, da er mit dieser gekoppelt ist. Insofern befindet sich der Magnetteller 83 bei den Betriebsstellungen 60 und 61 in Kontakt mit dem zweiten bestromten Elektrohaltemagnet 81, während er bei der Betriebsstellung 62 von dem unbestromten zweiten Elektrohaltemagnet 81 entfernt angeordnet ist.

Ist nun ein Infusionsspritzenwechsel gewünscht bzw. erforderlich, wird die Infusion gestoppt. Anschließend wird der Bedienhebel 34 am Antriebskopf 3 betätigt. Mit dem Betätigen des Bedienhebels 34 wird über den Haltearm 20, welcher als Rohr 90 konzipiert ist, via einer inneren Fixierungswelle 91 die Feder 92 belastete Freigabehülse 56 in Richtung des zweiten Elektrohaltemagneten 81 bewegt. Dieses Betätigen wird zusätzlich durch einen einen hier nicht gezeigten Sensor umfassenden Mikroschalter detektiert, wodurch die beiden Elektrohaltemagnete 80 und 81 über entsprechende Elektrokabel 93 bzw. 94 bestromt und die beiden Schiebehülsen 53 und 56 voneinander getrennt werden. Dabei öffnen sich die beiden Mutterschalen 12 und 13. Auch die Fixierbügel 95 bzw. 96 der Haltekralle 23 öffnen sich aufgrund der Betätigung des Bedienhebels 34. Der Formschluss zwischen der Vortriebsspindel 8 und der Vortriebsspindelmutter 9 ist gemäß der ersten Betriebsstellung 60 aufgehoben und der Antriebskopf 3 kann entgegen der Kolbenantriebsrichtung 10 in eine ausgefahrene Stellung (hier nicht beziffert) gebracht werden.

Anschließend wird der Klemmbügel 45 geöffnet und entsprechend verschwenkt, sodass die Aufnahme 44 gut zugänglich ist. Im Inneren des Infusionspumpengehäuses 40 erkennt ein Potentiometer 97 den Zustand des Klemmbügels 45. Jetzt kann die auszuwechselnde Infusionsspritze 5 entnommen und die neue Infusionsspritze 5 in die Aufnahme eingelegt werden. Die Infusionsspritzenflügel der einzulegenden Infusionsspritze 5 werden hierbei an der Klemmlasche 46 festgelegt, sodass die neue Infusionsspritze 5 an der Infusionsspritzenpumpe 2 axial fixiert ist. In diesem Zusammenhang wird auch der Klemmbügel 45 wieder geschlossen, sodass die Infusionsspritze 5 auch radial fixiert ist. Mittels des Potentiometers 97 am Klemmbügel 45 wird sogleich der Infusionsspritzendurchmesser gemessen.

Nun kann der Antriebskopf 3 entsprechend des Füllstands der Infusionsspritze 5 und damit auch der Position des Kolbens 4 an die Kolbenplatte 22 heran geführt werden. Damit hierbei größere Abstände zwischen dem Antriebskopf 3 und der Kolbenplatte 22 schneller überwunden werden können, kann dies durch eine händische Zustellbewegung in Kolbenantriebsrichtung 10 geschehen, da die beiden radial bewegbaren Mutterschalen 12 und 13 sich nicht im Eingriff mit dem Außengewinde 15 der Vortriebsspindel 8 befinden, wie dies bei der ersten Betriebsstellung 60 gemäß der Figuren 3A und 3B dargestellt ist. Bei der ersten Betriebsstellung 60 sind die beiden Elektrohaltemagnete 80 und 81 bestromt, die Schiebehülsen 53 und 56 voneinander getrennt angeordnet, die beiden Mutterschalen 12 und 13 radial geöffnet. Außerdem ist hierbei die Haltekralle 23 am Antriebskopf 3 geöffnet.

Sobald jedoch der Membranteller 21 mit der Kolbenplatte 22 in Kontakt tritt, bewegt sich insbesondere das Geberelement 27 nach innen in den Antriebskopf 3 hinein. Im Inneren des Antriebskopfgehäuses 24 durchfährt das Geberelement 27 nacheinander die zwei Lichtschranken 29 und 30 der Zwei-Schaltschwellen-Einrichtung 28.

Beim Durchfahren der ersten Lichtschranke 29 wird die Bestromung des ersten Elektrohaltemagneten 80 unterbrochen und die Auslösehülse 53 wird mittels der weiteren Feder 92 über die Rampen 72 der beiden radial bewegbaren Mutterschalen 12 und 13 geschoben, wodurch die Vortriebsspindelmutter 9 mit den Eingreifbereichen 64 in das Außengewinde 15 der Vortriebsspindel 8 formschlüssig eingreift. Hierbei blockiert die Vortriebseinrichtung 6, wie dies bei der zweiten Betriebsstellung 61 gemäß der Figuren 4A und 4B dargestellt ist. Eine händische Zustellbewegung in Kolbenantriebsrichtung 10 wird hierdurch schlagartig unterbunden.

Jedoch fährt die Vortriebseinrichtung 6 automatisch weiter, bis das Geberelement 27 die zweite Lichtschranke 30 durchfährt. Die zweite Lichtschranke 30 ist hierbei notwendig, da die Positionsveränderung der geschlossenen Mutterschalen 12 und 13 mit ihren Eingreifbereichen 64 gegenüber der Vortriebsspindel 8 abhängig ist, von der Reaktionsgeschwindigkeit des ersten Elektrohaltemagneten 80 und der Bewegungsgeschwindigkeit des Antriebskopfes 3 bis zur Kolbenplatte 22. Weiterhin besteht die Möglichkeit, dass die beiden radial bewegbaren Mutterschalen 12 und 13 beim Schließen der Vortriebsspindelmutter 9 lediglich auf den Gewindeflanken des Außengewindes 15 aufliegen und der Eingreifbereich 64 der Vortriebsspindelmutter 9 somit nicht wirkungsvoll in das Außengewinde 15 eingegriffen hat. Jedoch kann durch ein kurzes Verfahren bis zur zweiten Lichtschranke 30 die Vortriebsspindelmutter 9 ordnungsgemäß in die Vortriebsspindel 8 eingreifen. Mit dem Erreichen der zweiten Lichtschranke 30 ist die lineare Position der Antriebsvorrichtung 1 dann definiert. Bei dieser Referenzfahrt im Sinne der Erfindung befindet sich die Vortriebseinrichtung 6 in der zweiten Betriebsstellung 61, bei welcher der erste Elektrohaltemagnet 80 stromlos, der zweite Elektrohaltemagnet 81 jedoch bestromt ist. Beide Schiebehülsen 53 und 56 sind im Vortriebsschlitten 7 nach links verschoben und die beiden Mutterschalen 12 und 13 sind radial geschlossen. Die Haltekralle 23 am Antriebskopf 3 ist noch geöffnet.

Der Antriebskopf 3 fährt den abgeglichenen Weg von der zweiten Lichtschranke 30 bis zu einem Kraftsensor (hier nicht explizit gezeigt), mittels welchem ein korrektes Anliegen der Kolbenplatte 22 an dem Antriebskopf 3 detektiert und signalisiert wird.

Der zweite Elektrohaltemagnet 81 wird stromlos geschaltet, die Freigabehülse 56 verlagert sich in ihre Schließposition 77, die Haltekralle 23 schließt sich und die Fixierbügel 95 und 96 legen sich form- und kraftschlüssig an die Kolbenplatte 22 der Infusionsspritze 5 an. Die Infusionsspritze 5 ist nun sicher und spielfrei gefasst. Mittels des Klemmbügels 45 und dem Potentiometer 97 ist bereits der Infusionsspritzendurchmesser gemessen worden, sodass in einem Display (hier nicht gezeigt) der Infusionspumpe 2 eine Auswahl an entsprechenden Infusionsspritzen 5 vorgeschlagen wird. Nach einer Bestätigung der gültigen Infusionsspritze 5 kann die gewünschte Infusionstherapie eingegeben und es kann nun die Infusion gestartet werden, wobei sich die Vortriebseinrichtung 6 in der dritten Betriebsstellung 62 gemäß der Figuren 5A und 5B befindet. In der dritten Betriebsstellung 62 ist die Vortriebseinrichtung 6 somit verriegelt. Dies bedeutet, dass die beiden Elektrohaltemagnete 80, 81 stromlos sind. Besonders vorteilhaft ist es, dass hierbei ein energieloser Zustand hinsichtlich der beiden Elektrohaltemagnete 80, 81 vorliegt, sodass ein Funktionieren der dritten Betriebsstellung 62 auch ohne Bestromen der Elektrohaltemagnete 80, 81 vorliegt. Die beiden Schiebehülsen 53, 56 sind innerhalb des Vortriebsschlittens 7 nach rechts verschoben, da die weitere Feder 92 stärker ausgelegt ist als die Spiralfeder 55. Die beiden Mutterschalen bleiben radial verschlossen sowie auch die Haltekralle 23 am Antriebskopf 3 ebenfalls verschlossen ist.

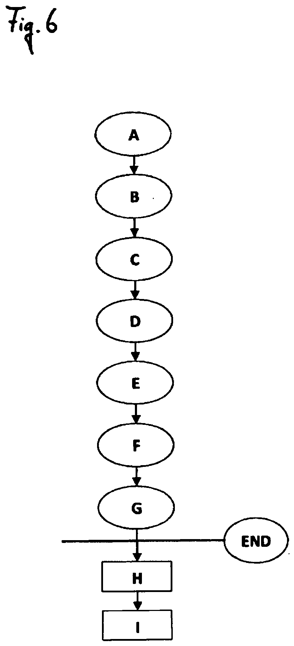

In der Darstellung gemäß der Figur 6 ist ein bevorzugter Prozessablauf hinsichtlich der erfindungsgemäßen Antriebsvorrichtung 1 illustriert. Er beginnt vorzugsweise mit dem Schritt A, bei welchem die Vortriebsspindelmutter 9 und die Haltekralle 23 manuell geöffnet werden. Es folgt Schritt B, bei welchem die Haltekralle 23 in ihrer geöffneten Position gemäß der Darstellung der Figur 1 einrastet. Hinsichtlich Schritts C bleibt die Vortriebsspindelmutter 9 durch die beiden Elektrohaltemagnete 80 und 81 in ihrer geöffneten Position. Anschließend wird der Antriebskopf 3 mit dem Schritt D in gewünschter Weise händisch verschoben. Im Schritt E wird der Membranteller 21 händisch auf die Kolbenplatte 22 aufgefahren. Gemäß Schritt F wird die erste Lichtschranke 29 mittels des vorlaufenden Geberelements 27 betätigt, wobei der Schaltpunkt ca. 3,5 mm vor der Kolbenplatte 22 liegt. Im Schritt G schließt ein Signal der ersten Lichtschranke 29 die Vortriebspindelmutter 9 radial durch Ausschalten des ersten Elektrohaltemagneten 80 und die Blockiereinrichtung 51 blockiert eine weitere händische Zustellbewegung des Antriebskopfes 3. An dieser Stelle ist der eigentliche Prozess zur Verhinderung einer unbeabsichtigten Bolusgabe abgeschlossen. Es folgt noch Schritt H, bei welchem der Antriebskopf 3 unter Zuhilfenahme der zweiten Lichtschranke 30 nun automatisch weiter auf die Kolbenplatte 22 vorwärtsbewegt wird. Und im Schritt I schließt sich die Haltekralle 23 wieder.

Es versteht sich, dass es sich bei dem vorstehend erläuterten Ausführungsbeispiel lediglich um eine erste Ausgestaltung der erfindungsgemäßen Antriebsvorrichtung handelt. Insofern beschränkt sich die Ausgestaltung der Erfindung nicht auf dieses Ausführungsbeispiel.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Antriebsvorrichtung
- 2: Infusionspumpe
- 3: Antriebskopf
- 4: Kolben
- 5: Infusionsspritze
- 6: Vortriebseinrichtung
- 7: Vortriebsschlitten
- 8: Vortriebsspindel
- 9: mehrteilige Vortriebsspindelmutter
- 10: Kolbenantriebsrichtung
- 11: Längserstreckung
- 12: erste Mutterschale
- 13: zweite Mutterschale
- 14: radiale Bewegungsrichtung
- 15: Außengewinde
- 20: Haltearm
- 21: Membranteller
- 22: Kolbenplatte
- 23: Haltekralle
- 24: Antriebskopfgehäuse
- 25: Kolbenplattensensor
- 26: Feder
- 27: Geberelement
- 28: Zwei-Schaltschwellen-Einrichtung
- 29: erste Lichtschranke
- 30: zweite Lichtschranke
- 31: Antriebsgetriebe
- 32: Signalleitung
- 33: Steuerung
- 34: Bedienhebel
- 40: Infusionspumpengehäuse
- 41: Regel- und/oder Steuereinrichtungen
- 42: Sensor zum Detektieren
- 43: Formschlusssensor
- 44: Aufnahme
- 45: Klemmbügel
- 46: Klemmlasche
- 50: Sicherungsvorrichtung
- 51: Blockiereinrichtung
- 52: Betätigungselement
- 53: Auslösehülse
- 54: Beschleunigungsmittel
- 55: Spiralfeder bzw. Auslösehülsefeder
- 56: Freigabehülse
- 57: Längserstreckung
- 60: erste Betriebsstellung
- 61: zweite Betriebsstellung
- 62: dritte Betriebsstellung
- 64: Eingreifbereich
- 65: Auflagebereich
- 66: Kippachse
- 68: Vortriebsschlittenlager
- 69: Kopfbereich
- 70: Fußbereich
- 71: Vortriebsspindelmuttergebiet
- 72: Rampe
- 73: Gegenlager
- 74: Vortriebsspindelmutterende
- 75: Parkeinrichtung
- 76: Öffenposition
- 77: Schließposition
- 78: weitere Öffenposition
- 79: weitere Schließposition
- 80: erster Elektrohaltemagnet bzw. Auslösehülsemagnet
- 81: zweiter Elektrohaltemagnet bzw. Freigabehülsemagnet
- 82: erster Magnetteller
- 83: zweiter Magnetteller
- 90: Rohr
- 91: Fixierungswelle
- 92: weitere Feder bzw. Freigabehülsefeder
- 93: erstes Elektrokabel
- 94: zweites Elektrokabel
- 95: erster Fixierbügel
- 96: zweiter Fixierbügel
- 97: Potentiometer

## Patentansprüche

1. Antriebsvorrichtung (1) mit einem Infusionsspritzenkolbenantriebskopf (3) zum Bewegen eines Infusionsspritzenkolbens (4) umfassend eine Vortriebseinrichtung (6) mit einem Vortriebsschlitten (7), mit einer Vortriebsspindel (8) und mit einer wenigstens eine radial bewegbare Mutterschale (12, 13) aufweisenden mehrteiligen Vortriebsspindelmutter (9) und umfassend eine mittels eines Kontrollsensors (25) automatisch auslösbare Blockiereinrichtung (51) zum Blockieren einer Zustellbewegung des Antriebskopfes (3), bei welcher der Vortriebsschlitten (7) mittels der Vortriebsspindelmutter (9) an der Vortriebsspindel (8) treibbar ist und bei welchem der Antriebskopf (3) an dem Vortriebsschlitten (7) angeordnet ist,
**dadurch gekennzeichnet, dass**
die mittels des Kontrollsensors (25) automatisch auslösbare Blockiereinrichtung (51) die mehrteilige Vortriebsspindelmutter (9) zum Blockieren der Zustellbewegung des Antriebskopfes (3) an der Vortriebsspindel (8) umfasst.

2. Antriebsvorrichtung (1) nach Anspruch 1,
**dadurch gekennzeichnet, dass**
die Blockiereinrichtung (51) eine axial verschiebliche Auslösehülse (53) umfasst, welche die Vortriebsspindelmutter (9) radial außen umgibt.

3. Antriebsvorrichtung (1) nach Anspruch 1 oder 2,
**dadurch gekennzeichnet, dass**
die Blockiereinrichtung (51) Mittel zum Beschleunigen einer Auslösehülse (53) umfasst, wobei die Beschleunigungsmittel (54) insbesondere eine Beschleunigungsspiralfeder (55) aufweisen, welche radial außen auf der Auslösehülse (53) angeordnet ist.

4. Antriebsvorrichtung (1) nach einem der Ansprüche 1 bis 3,
**dadurch gekennzeichnet, dass**
die Blockiereinrichtung (51) mittels einer Schiebehülse (53) oder vorzugsweise wenigstens zwei Schiebhülsen (53, 56) steuerbar ist, wobei die wenigstens zwei 5 Schiebehülsen (53, 56) einzeln antreibbar und/oder sequentiellverschiebbar angeordnet sind.

5. Antriebsvorrichtung (1) nach einem der Ansprüche 1 bis 4,
**dadurch gekennzeichnet, dass**
die wenigstens eine radial bewegbare Mutterschale (12,13) ein in Bezug auf ihre Längserstreckung (57) axial verschiebliches Betätigungselement (52) aufweist, welches in Bezug auf die Vortriebsspindel (8) ein radiales Betätigen der wenigstens einen radial bewegbaren Mutterschale (12, 13) bewirkt.

6. Antriebsvorrichtung (1) nach einem der Ansprüche 1 bis 5,
**dadurch gekennzeichnet, dass**
die wenigstens eine radial bewegbare Mütterschale (12, 13) radial außen von wenigstens einer Schiebehülse (53), vorzugsweise von zwei Schiebehülsen (53, 56), zumindest teilweise umgeben ist, wobei die Wenigstens eine radial bewegbare Mutterschale (12, 13) von wenigstens einer Schiebehülse (53), vorzugsweise von zwei Schiebehülsen (53, 56), radial verlagerbar ist.

7. Antriebsvorrichtung (1) nach einem derAnsprüche 1 bis 6,
**dadurch gekennzeichnet, dass**
die wenigstens eine radial bewegbare Mutterschale (12, 13) einen Eingreifbereich (64) zum Eingreifen in ein Außengewinde (15) der Vortriebsspindel (8) und einen Auflagebereich (65) zum radialen Aufliegen auf das Außengewinde (15) der Vortriebsspindel (8) aufweist, wobei der Eingreifbereich (64) und der Auflagebereich (65) axial hintereinander angeordnet sind.

8. Antriebsvorrichtung (1) nach einem der Ansprüche 1 bis 7,
**dadurch gekennzeichnet, dass**
die wenigstens eine radial bewegbare Mutterschale (12, 13) endseitig (67) auf einem Vortriebsschlittenlager (68) gelagert ist.

9. Antriebsvorrichtung (1) nach einem der Ansprüche 1 bis 8,
**dadurch gekennzeichnet, dass**
die wenigstens eine radial bewegbare Mutterschale (12, 13) im Wesentlichen quer zu ihrer Längserstreckung (57) kippbar gegenüber der Vortriebsspindel (8) angeordnet ist.

10. Antriebsvorrichtung (1) nach einem der Ansprüche 1 bis 9,
**dadurch gekennzeichnet, dass**
die Vortriebspindelmutter (9) einen Kopfbereich (69) und einen Fußbereich (70) aufweist, deren jeweilige Außendurchmesser größer sind als ein zwischen dem Kopf- und Fußbereich (69, 70) angeordnetes Vortriebspindelmuttergebiet (71).

11. Antriebsvorrichtung (1) nach einem der Ansprüche 1 bis 10,
**dadurch gekennzeichnet, dass**
der Kopfbereich (69) eine Rampe (72) für axial verschiebliche Schiebehülsen (53, 56) und der Fußbereich (70) ein Gegenlager (73) für wenigstens eine der axial verschieblichen Schiebehülsen (53, 56) ausgestaltet

12. Antriebsvorrichtung (1) nach einem der Ansprüche 1 bis 11,
**dadurch gekennzeichnet, dass**
die Vortriebseinrichtung (8) eine Parkeinrichtung (75) zum zwischenzeitlichen Parken einer Schiebehülse (56) aufweist, welche axial neben der Vortriebsspindelmutter (9) angeordnet ist.

13. Antriebsvorrichtung (1) nach einem der Ansprüche 1 bis 12,
**dadurch gekennzeichnet, dass**
die Vortriebseinrichtung (8) insbesondere zum Halten der Vortriebsspindelmutter (9) in einer geöffneten Betriebsstellung (60) steuerbare Elektrohaltemagnete (80, 81) umfasst.

14. Infusionspumpe (2) mit einem Antriebskopf (3) zum Bewegen eines Infusionsspritzenkolbens (4) einer an der Infusionspumpe (2) angeordneten Infusionsspritze (5), mit einer Vortriebseinrichtung (6) zum Treiben des Antriebskopfes (3) und mit einer Sicherungsvorrichtung (50) zum Verhindern einer unbeabsichtigten Bolusgabe,
**dadurch gekennzeichnet, dass**
die Infusionspumpe (2) eine Antriebsvorrichtung (1) nach einem der vorhergehenden Ansprüche aufweist.

15. Verfahren zum Betreiben einer Infusionspumpe (2), bei welchem ein Antriebskopf (3) für einen Infusionsspritzenkolben (4) einer Infusionsspritze (5) mittels einer Vortriebseinrichtung (6) vorwärts getrieben wird, bei welchem zum Treiben des Antriebskopfes (3) eine mehrteilige Vortriebsspindelmutter (9) entlang einer Vortriebsspindel (8) bewegt wird und bei welchem ein Betätigungselement (52) einer Sicherungsvorrichtung (50) zum Verhindern einer unbeabsichtigten Bolusgabe ausgelöst wird, wenn der Antriebskopf (3) bei einem Infusionsspritzenwechsel bis an den infusionsspritzenkolben (4) heranbewegt wird,
**dadurch gekennzeichnet, dass**
das Betätigungselement (52) axial entlang der Vortriebsspindel (8) verschoben wird, wodurch die mehrteilige Vortriebsspindelmutter (9) zumindest teilweise radial auf die Vortriebsspindel (8) zubewegt oder radial von der Vortriebsspindel (8) fortbewegt wird.

## Claims

1. A drive device (1) with an infusion syringe piston drive head (3) for moving an infusion syringe piston (4) comprising an advancing means (6) including an advancing slide (7), an advancing spindle (8) and a multi-part advancing spindle nut (9) including at least one radially movable nut shell (12, 13) and comprising a blocking means (51) adapted to be automatically triggered by means of a control sensor (25) for blocking an infeed motion of the drive head (3) in which the advancing slide (7) can be driven on the advancing spindle (8) by means of the advancing spindle nut (9) and in which the drive head (3) is arranged on the advancing slide (7),
**characterized in that**
the blocking means (51) adapted to be automatically triggered by means of the control sensor (25) comprises the multi-part advancing spindle nut (9) for blocking the infeed motion of the drive head (3) on the advancing spindle (8).

2. The drive device (1) according to claim 1,
**characterized in that**
the blocking means (51) comprises an axially displaceable trigger sleeve (53) which surrounds the advancing spindle nut (9) on the radial outside.

3. The drive device according to claim 1 or 2,
**characterized in that**
the blocking means (51) comprises means for accelerating a trigger sleeve (53), the accelerating means (54) especially including an accelerating coil spring (55) which is arranged radially outside on the trigger sleeve (53).

4. The drive device (1) according to any one of the claims 1 to 3,
**characterized in that**
the blocking means (51) is controllable by means of one sliding sleeve (53) or preferably at least two sliding sleeves (53, 56), wherein the at least two sliding sleeves (53, 56) are arranged to be individually drivable and/or to be sequentially movable.

5. The drive device (1) according to any one of the claims 1 to 4,
**characterized in that**
the at least one radially movable nut shell (12, 13) includes an actuating element (52) axially displaceable with respect to its longitudinal extension (57) which causes radial actuation of the at least one radially movable nut shell (12, 13) with respect to the advancing spindle (8).

6. The drive device (1) according to any one of the claims 1 to 5,
**characterized in that**
the at least one radially movable nut shell (12, 13) is at least partly surrounded radially outside by at least one sliding sleeve (53), preferably by two sliding sleeves (53, 56), wherein the at least one radially movable nut shell (12, 13) is radially displaceable by at least one sliding sleeve (53), preferably by two sliding sleeves (53, 56).

7. The drive device (1) according to any one of the claims 1 to 6,
**characterized in that**
the at least one radially movable nut shell (12, 13) includes an engaging area (64) for engaging in a male thread (15) of the advancing spindle (8) and a supporting area (65) for radially bearing on the male thread (15) of the advancing spindle (8), wherein the engaging area (64) and the supporting area (65) are arranged axially in series.

8. The drive device (1) according to any one of the claims 1 to 7,
**characterized in that**
the at least one radially movable nut shell (12, 13) is supported at the end side (67) on an advancing slide bearing (68).

9. The drive device (1) according to any one of the claims 1 to 8,
**characterized in that**
the at least one radially movable nut shell (12, 13) is arranged to be tilting vis-a-vis the advancing spindle (8) substantially transversely to its longitudinal extension (57).

10. The drive device (1) according to any one of the claims 1 to 9,
**characterized in that**
the advancing spindle nut (9) has a head area (69) and a foot area (70) the respective outer diameters of which are larger than an advancing spindle nut zone (71) disposed between the head and foot areas (69, 70).

11. The drive device (1) according to any one of the claims 1 to 10,
**characterized in that**
the head area (69) configures a ramp (72) for axially displaceable sliding sleeves (53, 56) and the foot area (70) configures a counter-bearing (73) for at least one of the axially displaceable sliding sleeves (53, 56).

12. The drive device (1) according to any one of the claims 1 to 11,
**characterized in that**
the advancing means (8) includes a parking means (75) for intermediately parking a sliding sleeve (56) arranged axially adjacent to the advancing spindle nut (9).

13. The drive device (1) according to any one of the claims 1 to 12,
**characterized in that**
the advancing means (8) comprises controllable holding solenoids (80, 81) especially for maintaining the advancing spindle nut (9) in an opened operating position (60).

14. An infusion pump (2) comprising a drive head (3) for moving an infusion syringe piston (4) of an infusion syringe (5) arranged on an infusion pump (2), comprising an advancing means (6) for driving the drive head (3) and comprising a safety device (50) for preventing inadvertent bolus administration,
**characterized in that**
the infusion pump (2) includes a drive device (1) in accordance with any one of the preceding claims.

15. A method of operating an infusion pump (2) in which a drive head (3) for an infusion syringe piston (4) of an infusion syringe (5) is advanced by an advancing means (6), in which a multi-part advancing spindle nut (9) is moved along an advancing spindle (8) for driving the drive head (3) and in which an actuating element (52) triggers a safety device (50) for preventing inadvertent bolus administration, when the drive head (3) is moved up to the infusion syringe piston (4) during replacement of the infusion syringe,
**characterized in that**
the actuating element (52) is axially displaced along the advancing spindle (8), thereby the multi-part advancing spindle nut (9) being radially moved at least partly toward the advancing spindle (8) or being radially moved away from the advancing spindle (8).

## Revendications

1. Dispositif d'entraînement (1) avec une tête d'entraînement de piston de seringue de perfusion (3) pour le déplacement d'un piston de seringue de perfusion (4) comprenant un dispositif de propulsion (6) avec un chariot de propulsion (7), avec une broche de propulsion (8) et avec un écrou de broche de propulsion (9) en plusieurs parties comprenant au moins une coque d'écrou (12, 13) mobile radialement et comprenant un dispositif de blocage (51) pouvant être déclenché automatiquement au moyen d'un capteur de contrôle (25) pour le blocage d'un mouvement d'avance de la tête d'entraînement (3), dans lequel le chariot de propulsion (7) peut être entraîné au moyen de l'écrou de broche de propulsion (9) sur la broche de propulsion (8) et dans lequel la tête d'entraînement (3) est disposée sur le chariot de propulsion (7),
**caractérisé en ce que**
le dispositif de blocage (51) pouvant être déclenché automatiquement au moyen du capteur de contrôle (25) comprend l'écrou de broche (9) en plusieurs parties pour le blocage du mouvement d'avance de la tête d'entraînement (3) sur le broche de propulsion (8).

2. Dispositif d'entraînement (1) selon la revendication 1,
**caractérisé en ce que**
le dispositif de blocage (51) comprend un manchon de déclenchement (53) mobile axialement, qui entoure de manière externe radialement l'écrou de broche de propulsion (9).

3. Dispositif d'entraînement (1) selon la revendication 1 ou 2,
**caractérisé en ce que**
le dispositif de blocage (51) comprend des moyens pour l'accélération du manchon de déclenchement (53), les moyens d'accélération (54) comprenant plus particulièrement un ressort en spirale d'accélération (55), qui est disposé radialement à l'extérieur sur le manchon de déclenchement (53).

4. Dispositif d'entraînement (1) selon l'une des revendications 1 à 3,
**caractérisé en ce que**
le dispositif de blocage (51) peut être contrôlé au moyen d'un manchon de poussée (53) ou de préférence d'au moins deux manchons de poussée (53, 56), les au moins deux manchons de poussée (53, 56) pouvant être entraînés individuellement et/ou étant disposés de manière mobile séquentiellement.

5. Dispositif d'entraînement (1) selon l'une des revendications 1 à 4,
**caractérisé en ce que**
l'au moins une coque d'écrou (12, 13) mobile radialement comprend un élément d'actionnement (52) mobile axialement par rapport à son extension longitudinale (57), qui provoque, par rapport à la broche de propulsion (8), un actionnement radial de l'au moins une coque d'écrou (12, 13) mobile axialement.

6. Dispositif d'entraînement (1) selon l'une des revendications 1 à 5,
**caractérisé en ce que**
l'au moins une coque d'écrou (12, 13) mobile radialement est entourée radialement à l'extérieur au moins partiellement par au moins un manchon de poussée (53), de préférence par deux manchons de poussée (53, 56), l'au moins une coque d'écrou (12, 13) mobile radialement pouvant être déplacée radialement par au moins un manchon de poussée (53), de préférence par deux manchons de poussée (53, 56).

7. Dispositif d'entraînement (1) selon l'une des revendications 1 à 6,
**caractérisé en ce que**
l'au moins une coque d'écrou (12, 13) mobile radialement comprend une zone d'emboîtement (64) pour l'emboîtement dans un filetage externe (15) de la broche de propulsion (8) et une zone d'appui (65) pour l'appui radial sur le filetage externe (15) de la broche de propulsion (8), la zone d'emboîtement (64) et la zone d'appui (65) étant disposée axialement l'une derrière l'autre.

8. Dispositif d'entraînement (1) selon l'une des revendications 1 à 7,
**caractérisé en ce que**
l'au moins une coque d'écrou (12, 13) mobile radialement est logée, au niveau de son extrémité (67), sur un palier de chariot de propulsion (68).

9. Dispositif d'entraînement (1) selon l'une des revendications 1 à 8,
**caractérisé en ce que**
l'au moins une coque d'écrou (12, 13) mobile radialement est disposée de façon à pouvoir être basculée globalement transversalement par rapport à son extension longitudinale (57) par rapport à la broche de propulsion (8).

10. Dispositif d'entraînement (1) selon l'une des revendications 1 à 9,
**caractérisé en ce que**
l'écrou de broche de propulsion (9) comprend une zone de tête (69) et une zone de pied (70) dont les diamètres respectifs sont supérieurs à une zone d'écrou de broche de propulsion (71) disposée entre la zone de tête et la zone de pied (69, 70).

11. Dispositif d'entraînement (1) selon l'une des revendications 1 à 10,
**caractérisé en ce que**
la zone de tête (69) comprend une rampe (72) pour des manchons de poussée (53, 56) coulissants axialement et la zone de pied (70) comprend une butée (73) pour au moins un des manchons de poussée (53, 56) coulissants axialement.

12. Dispositif d'entraînement (1) selon l'une des revendications 1 à 11,
**caractérisé en ce que**
le dispositif de propulsion (8) comprend un dispositif de stationnement (75) pour le stationnement temporaire d'un manchon de poussée (56) qui est disposée axialement à côté de l'écrou de broche de propulsion (9).

13. Dispositif d'entraînement (1) selon l'une des revendications 1 à 12,
**caractérisé en ce que**
le dispositif de propulsion (8) comprend, plus particulièrement, pour le maintien de l'écrou de broche de propulsion (9) dans une position de fonctionnement ouverte (60), des électro-aimants (80, 81) contrôlables.

14. Pompe à perfusion (2) avec une tête d'entraînement (3) pour le déplacement d'un piston de seringue de perfusion (4) d'une seringue de perfusion (5) disposée sur la pompe à perfusion (2), avec un dispositif de propulsion (6) pour l'entraînement de la tête d'entraînement (3) et avec un dispositif de sécurisation (50) pour empêcher l'administration non souhaitée d'un bolus,
**caractérisé en ce que**
la pompe à perfusion (2) comprend un dispositif d'entraînement (1) selon l'une des revendications précédentes.

15. Procédé d'exploitation d'une pompe à perfusion (2), dans lequel une tête d'entraînement (3) pour un piston de seringue de perfusion (4) d'une seringue de perfusion (5) est entraînée vers l'avant au moyen d'un dispositif de propulsion (6), dans lequel, pour l'entraînement de la tête d'entraînement (3), un écrou de broche de propulsion (9) en plusieurs parties est déplacé le long d'une broche de propulsion (8) et dans lequel un élément d'actionnement (52) d'un dispositif de sécurisation (50) pour empêcher une administration de bolus non souhaitée est déclenché lorsque la tête d'entraînement (3) est déplacée lors d'un changement de seringue de perfusion jusqu'au piston de seringue de perfusion (4),
**caractérisé en ce que**
l'élément d'actionnement (52) est déplacé axialement le long de la broche de propulsion (8), ce qui déplace l'écrou de broche de propulsion (9) au moins partiellement radialement vers la broche de propulsion (8) ou continue son déplacement radial à partir de la broche de propulsion (8).
